(19) Europäisches Patentamt
European Patent Office
Office européen des brevets

(11) **EP 3 137 769 B1**

(12) **FASCICULE DE BREVET EUROPEEN**

(45) Date de publication et mention
de la délivrance du brevet:
**24.02.2021 Bulletin 2021/08**

(21) Numéro de dépôt: **15718932.5**

(22) Date de dépôt: **30.04.2015**

(51) Int Cl.:
*F04B 43/04* (2006.01)     *F04B 43/12* (2006.01)
*F04B 49/06* (2006.01)     *F04B 43/09* (2006.01)
*A61M 5/14* (2006.01)     *G01F 1/84* (2006.01)
*G01F 15/00* (2006.01)     *F04B 49/14* (2006.01)
*F04B 49/10* (2006.01)     *A61M 5/142* (2006.01)

(86) Numéro de dépôt international:
**PCT/EP2015/059516**

(87) Numéro de publication internationale:
**WO 2015/166051 (05.11.2015 Gazette 2015/44)**

(54) **SYSTEME DE MODULATION DE LA QUANTITE DE LIQUIDE DELIVRE PAR UNE MICRO-POMPE A COMMANDE PIEZO-ELECTRIQUE**

SYSTEM ZUR MODULIERUNG DER VON EINER MIKROPUMPE MIT PIEZOELEKTRISCHER STEUERUNG GEFÖRDERTEN FLÜSSIGKEITSMENGE

SYSTEM FOR MODULATION OF THE QUANTITY OF LIQUID DELIVERED BY A MICRO-PUMP WITH PIEZO-ELECTRIC CONTROL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.04.2014 FR 1453962**

(43) Date de publication de la demande:
**08.03.2017 Bulletin 2017/10**

(73) Titulaire: **Commissariat à l'Énergie Atomique et aux Énergies Alternatives
75015 Paris (FR)**

(72) Inventeurs:
• **CAMPAGNOLO, Raymond
F-38100 Grenoble (FR)**
• **FOUILLET, Yves
F-38340 Voreppe (FR)**
• **FUCHS, Olivier
F-38600 Fontaine (FR)**

(74) Mandataire: **Brevalex
95, rue d'Amsterdam
75378 Paris Cedex 8 (FR)**

(56) Documents cités:
WO-A1-03/027503        DE-A1- 10 238 600
DE-A1-102005 058 080    DE-A1-102008 042 071
FR-A- 1 200 165         FR-A1- 2 142 782
FR-A1- 2 989 590        US-A- 5 816 780

## Description

### DOMAINE TECHNIQUE ET ART ANTÉRIEUR

Le domaine de l'invention se rapporte à la maîtrise de la production d'une impulsion de volume liquide à partir d'une micro-pompe silicium comportant une ou des membrane(s) déformée(s) par des actionneurs piézo-électriques.

Des micropompes ont été précédemment développées, qui s'avèrent adapter à la délivrance de principes actifs dans le corps d'un animal ou d'un patient. La demande de brevet WO2011/058140 décrit par exemple une pompe particulièrement compacte, et adaptée à être implantée. La demande de brevet FR 2 989 590 A1 décrit par exemple un système d'injection localisée d'un fluide médical et/ou thérapeutique utilisant une pompe péristaltique.

L'expérience montre que le débit de liquide délivré par i telles pompes peut varier en fonction de la pression de fluide en aval de la pompe.

Or, dans certaines applications, le débit de liquide délivré par la pompe doit pouvoir être maîtrisé. L'invention se propose de résoudre ce problème.

### EXPOSÉ DE L'INVENTION

L'invention concerne d'abord un système d'injection de liquide, ou un système pour délivrer un liquide, comportant :

- une pompe péristaltique, fonctionnant par cycle, de telle sorte qu'un volume élémentaire de liquide est délivré dans le conduit au cours de chaque cycle,
- au moins un actionneur apte à provoquer la délivrance d'un volume de liquide de la pompe, au cours d'un cycle,
- un circuit de commande, pour appliquer un signal de commande à l'actionneur, de telle sorte que le volume délivré au cours d'un cycle dépend de ce signal appliqué à l'actionneur,
- un capteur, apte à produire un signal représentant la quantité de liquide délivrée au cours d'un cycle,
- le circuit de commande étant apte à déterminer un signal de commande en fonction dudit signal du capteur et d'un signal de consigne.

La pompe peut comporter une chambre centrale dont le volume peut varier sous l'effet de la déformation ou du déplacement d'une de ses parois, cette variation entraînant la délivrance dudit volume élémentaire.

2 vannes peuvent être, disposées de part et d'autre de cette chambre centrale.

Selon une réalisation, la chambre centrale peut comporter une membrane, dont la déformation est apte à faire varier le volume de ladite chambre centrale, et dont le déplacement est commandé par un desdits actionneurs.

Le capteur peut, selon un mode de réalisation, être apte à mesurer une déformation ou un déplacement de la membrane, le système comportant en outre des moyens pour calculer la quantité de liquide délivré au cours d'un cycle, par exemple par une estimation de type calotte sphérique.

En variante, le capteur peut comporter un débitmètre, interne ou externe à la pompe, associé à des moyens pour intégrer le signal de ce débitmètre, fournissant ainsi un signal représentatif du volume délivré au cours d'un cycle. Le capteur peut être disposé en aval ou en amont de la pompe, les termes amont et aval se référant au sens d'écoulement du fluide pompé.

Dans une réalisation particulière, le capteur est apte à délivrer un signal représentant la quantité moyenne de liquide délivrée au cours d'un cycle, ainsi qu'au cours d'un ou plusieurs cycles précédent ledit cycle.

L'actionneur peut être piézoélectrique.

Un système selon l'invention peut comporter au moins une alimentation en tension apte à piloter l'actionneur, cette alimentation étant apte à être commandée par le signal de commande. Ce dernier peut être un signal de modulation de l'amplitude ou de la fréquence, de la tension.

Dans le cas d'une modulation de la fréquence, celle-ci peut être réalisée à une fréquence de quelques dizaines de kHz, par exemple entre 10 kHz et 50 kHz, ou même encore jusqu'à 500 kHz.

Dans une réalisation, des moyens sont prévus, pour générer un signal de commande qui est, tant que la quantité de liquide délivrée, ou le volume débité, est inférieur(e) à une valeur de consigne, une fonction croissante en fonction du temps, par exemple en forme de rampe. Par exemple, le système peut comporter un générateur de courant qui charge une capacité, générant ainsi ladite rampe.

L'invention concerne également un procédé de mise en œuvre d'un dispositif selon l'invention, en particulier tel que décrit ci-dessus, pour délivrer un volume unitaire de fluide au cours d'un cycle de la pompe.

### BRÈVE DESCRIPTION DES DESSINS

- les figures 1A- 1F représentent des étapes de fonctionnement d'une micro pompe utilisée dans le cadre d'une réalisation selon la présente invention,
- la figure 2 représente l'évolution du volume élémentaire expulsé par la pompe en fonction de la tension d'actionnement (en V) de la membrane,
- la figure 3 représente schématiquement un système de contrôle analogique du débit,
- la figure 4 représente schématiquement un système de contrôle numérique du débit, mettant en œuvre un débitmètre,
- la figure 5 représente schématiquement un système de contrôle analogique d'un volume unitaire délivré,

- la figure 6 représente schématiquement un système de contrôle numérique du volume unitaire délivré, mettant en œuvre un débitmètre,
- la figure 7 représente schématiquement un système de contrôle avec modulation de type PWM de la haute tension.

## EXPOSÉ DÉTAILLÉ DE MODES DE RÉALISATION PARTICULIERS

D'une façon générale, l'invention concerne toute pompe présentant une chambre, dont le volume peut varier, au cours d'un cycle, sous l'effet de la déformation ou du déplacement d'une de ses parois, cette variation entraînant le refoulement (ou la délivrance) d'un volume élémentaire.

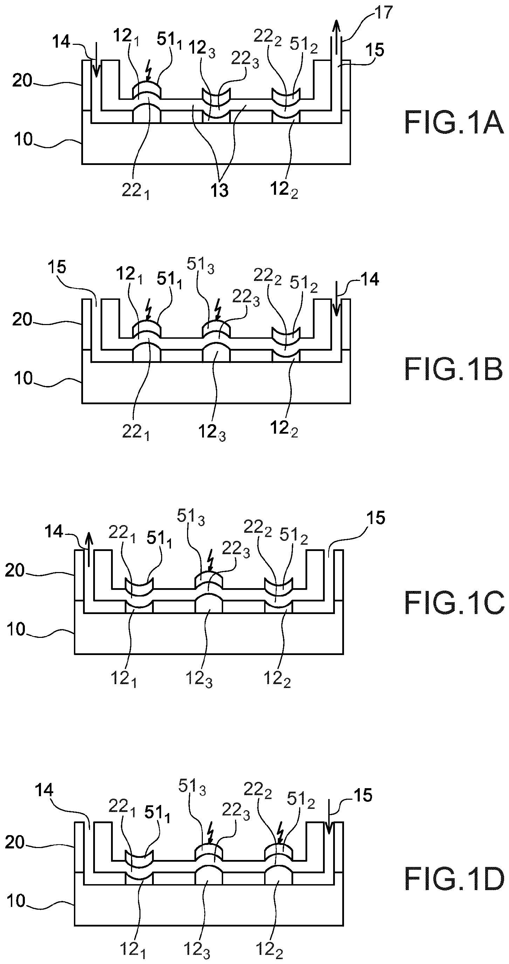

Un exemple de pompe 1 à laquelle l'invention peut être appliquée, et son procédé de fonctionnement, sont illustrés en figures 1A- 1F.

Cette pompe est une pompe à membrane, cette dernière étant apte à se déformer.

Elle est par exemple réalisée dans un premier substrat 10 et un second substrat 20, par exemple en silicium ou en verre, comme décrit dans la demande WO 2013/160312. D'autres détails de réalisation pourront être trouvés dans ce document.

La pompe comporte au moins 3 zones réalisées dans cet exemple dans le second substrat 20, pour former des membranes déformables $22_1$, $22_2$, $22_3$ (ici et dans la suite, les indices « 1 », respectivement « 2 », se rapportent à la chambre d'entrée, respectivement de sortie, de la pompe, l'indice « 3 » à la chambre dont les variations de volume permettent le refoulement d'un volume élémentaire).

Ces membranes $22_1$, $22_2$, $22_3$ déformables sont respectivement disposées en vis-à-vis de cavités réalisées dans le premier substrat 10, de façon à constituer des chambres $12_1$, $12_2$, $12_3$. Chaque membrane $22_1$, $22_2$, $22_3$ constitue une paroi de la cavité à laquelle elle est associée. Ainsi, la déformation de la membrane $22_1$, respectivement $22_2$, $22_3$, permet de faire varier le volume de la chambre $12_1$, respectivement $12_2$, $12_3$, à laquelle elle est associée.

Dans cet exemple, les trois chambres peuvent être identiques et chacune a, par exemple, un diamètre de 7,5 mm et une profondeur de 30 $\mu$m. Sont également représentés des actionneurs $51_1$, $51_2$, $51_3$, par exemple de type piézoélectrique, chacun étant associé à l'une des chambres. Chaque actionneur est apte à déformer la membrane déformable auquel il est associé, par exemple pour la faire passer d'une première position (position ouverte) à une deuxième position (position fermée ou semi fermée).

Des conduits d'entrée 14 et de sortie 15 qui traversent le premier substrat 10 sont réalisés sous forme de puits débouchant à l'intérieur, respectivement, des chambres amont $12_1$ et aval $12_2$.

La référence 17 désigne un conduit de sortie auquel le conduit de sortie 15 de la pompe est relié.

Les chambres sont connectées en série par l'intermédiaire d'un, ou de, conduit(s) de communication 13. La chambre centrale $12_3$ forme une chambre de pompage, les deux chambres $12_1$ et $12_2$ formant des vannes, respectivement amont et aval. En variante, ces vannes pourraient être remplacées par d'autres types de vannes.

La chambre centrale $12_3$, disposée entre les chambres d'entrée $12_1$ et de sortie $12_2$, est apte à passer d'un premier volume à un deuxième volume, sous l'effet de la déformation de la membrane $22_3$. Cette variation de volume détermine le volume élémentaire de fluide $V_{cycle}$ délivré au cours d'un cycle de pompage, comme décrit ci-après.

Comme indiqué ci-dessus, l'invention peut s'appliquer à toute pompe présentant une chambre, dont le volume peut varier, au cours d'un cycle, sous l'effet de la déformation ou du déplacement d'une de ses parois. Aussi, en variante à une chambre dont une paroi comporte une membrane apte à se déformer, la pompe peut présenter une chambre dont une paroi se déplace, prenant par exemple la forme d'un piston.

La pompe détaillée ci-dessus fonctionne en période de 6 phases selon le mode opératoire suivant.

En figure 1A (première phase de fonctionnement), le fluide entre par le conduit 14 et en sort par le conduit 15, les actionneurs $51_1$ $51_2$, $51_3$ étant activés de telle sorte que la vanne constituée par la chambre $12_1$ est ouverte, tandis que les chambres $12_2$ et $12_3$ sont fermées, la vanne de sortie étant donc fermée.

S'ensuivent ensuite les phases suivantes, illustrées en figures 1B - 1F :

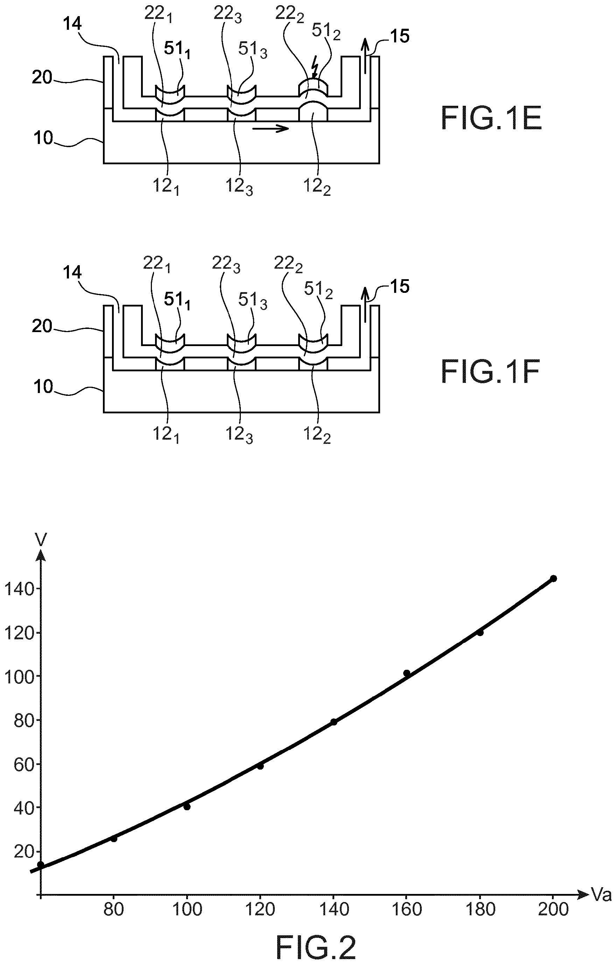

- activation des actionneurs $51_1$, $51_2$, $51_3$ de telle sorte que les chambres $12_1$, $12_2$ et $12_3$ soient, respectivement, ouverte, fermée et ouverte (figure 1B, phase 2); le fluide entre alors par le conduit 14 et circule de la première chambre $12_1$ vers la chambre centrale $12_3$,
- activation des actionneurs $51_1$ $51_2$, $51_3$ de telle sorte que les chambres $12_1$, $12_2$ et $12_3$ soient, respectivement, fermée, fermée et ouverte (figure 1C, phase 3); une partie minime du fluide est refoulée par le conduit d'entrée 14,
- activation des actionneurs $51_1$, $51_2$, $51_3$ de telle sorte que les chambres $12_1$, $12_2$ et $12_3$ soient, respectivement fermée, ouverte et ouverte (figure 1D, phase 4); une partie minime de fluide est aspirée du conduit de sortie 15,
- activation des actionneurs $51_1$ $51_2$, de telle sorte que les chambres $12_1$ et $12_2$, agissant en tant que vannes, soient, respectivement, fermée et ouverte et activation de l'actionneur central $51_3$ de manière à réduire le volume de la chambre centrale $12_3$ (figure 1E, phase 5). Le volume de fluide à délivrer est alors refoulé dans le canal de sortie 15. Un volume élémentaire est donc délivré, vers la sortie de la pompe, par l'actionnement de la membrane centrale. Le volume élémentaire délivré au cours du cycle correspond à la variation de volume de la chambre centrale $12_3$ durant cette phase.

- enfin, on active les actionneurs $51_1$ $51_2$, de telle sorte que les chambres $12_1$ et $12_2$, soient fermées. La chambre centrale $12_3$ reste telle qu'à l'issue de la phase précédente, le fluide continuant à être encore refoulé par le conduit de sortie 15 (figure 1F, phase 6).

Ainsi, le fonctionnement de cette pompe est cyclique, un volume élémentaire de fluide étant délivré (pompé) au cours de chaque cycle.

Dans une pompe de l'art antérieur, le volume élémentaire délivré est prédéterminé et constant et une variation du débit peut être obtenue par variation de la fréquence de fonctionnement de la pompe. Pour les très faibles débits, ou pour les débits moyens, on obtient donc un flux pulsé à très faible rapport cyclique, ce qui peut être difficilement acceptable.

On peut également obtenir un très faible débit, en particulier à une fréquence fixe, à condition de savoir ajuster le volume du volume élémentaire ; ceci est possible avec, par exemple, une pompe telle que celle décrite schématiquement ci-dessus, en ajustant la variation de volume de la chambre centrale $12_3$, ce qui s'obtient en contrôlant la déformation de la membrane $22_3$.

La courbe de la figure 2 représente un enregistrement temporel de l'évolution du volume élémentaire $V_{cycle}$ (en nanolitre) expulsé par la pompe en fonction de la tension d'actionnement $V_a$ (en Volt) de l'actionneur $51_3$ associé à la chambre centrale $12_3$. Cette courbe n'est pas parfaitement linéaire. On peut considérer que, si l'amplitude de la déformation de la membrane est linéaire avec la tension d'actionnement, alors le volume déplacé est celui d'une calotte sphérique. Une approximation conduit alors à une formule de la forme suivante :

$$V_{cycle}(V_a) = 0.056\ V_a^{3/2} - 14,$$

dans laquelle $V_a$ est la tension d'actionnement en Volt et $V_{cycle}$ est le volume élémentaire en nl.

Selon ce modèle, on peut considérer qu'il y a un lien entre la tension appliquée à l'actionneur $51_3$ de la chambre centrale $12_3$ et le volume élémentaire délivré au cours d'un cycle. Autrement dit, le signal de commande de l'actionneur central $51_3$ permet de contrôler déformation de la membrane, et, par conséquent, la variation du volume de la chambre centrale 12, et donc le volume élémentaire délivré au cours d'un cycle.

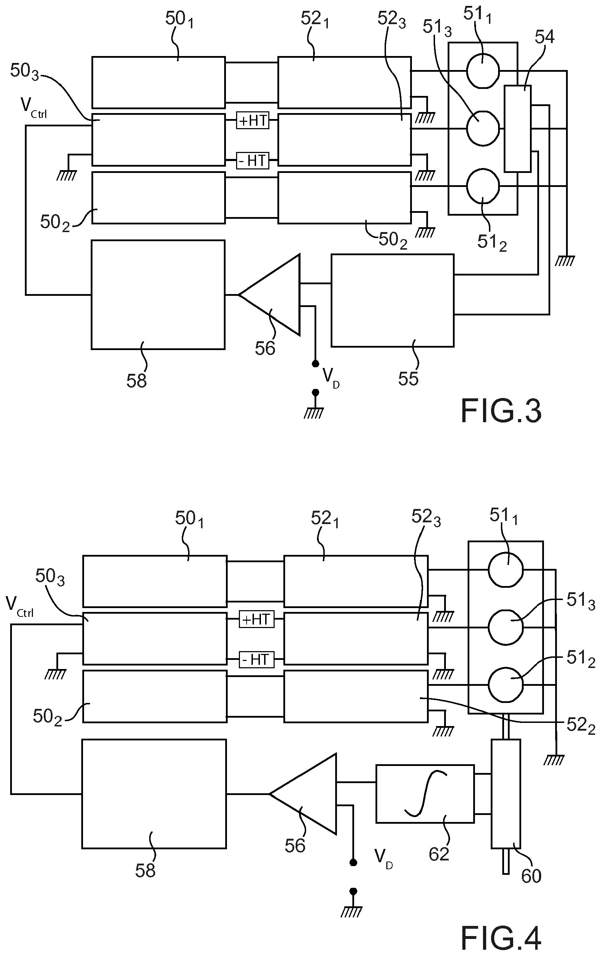

Un premier exemple de système d'asservissement selon l'invention est illustré en figure 3. Il peut être utilisé en combinaison avec une pompe du type décrit ci-dessus, mettant en œuvre trois actionneurs piézo-électriques $51_1$, $51_2$, $51_3$ (dans cet exemple, comme dans les suivants, les indices « 1 », respectivement « 2 », se rapporteront à l'actionneur d'entrée, respectivement de sortie, et aux éléments (circuit de commande, alimentation HT) correspondant à chacun de ces actionneurs, l'indice « 3 » se rapportant à l'actionneur central).

A chacun de ces actionneurs est associé un circuit de commande $52_1$, $52_2$,$52_3$, qui permet de produire les impulsions de commande de cet actionneur.

Chacun des circuits de commande $52_1$, $52_2$, $52_3$ est, à son tour, commandé par une alimentation haute tension $50_1$, $50_2$, $50_3$.

La tension délivrée par l'alimentation haute tension $50_1$, $50_2$ de chacun des circuits de commande d'entrée $52_1$, et de sortie $52_2$ est fixe. Les chambres $12_1$ et $12_2$ passent d'un état ouvert à un état fermé. Par contre, l'alimentation haute tension $50_3$ de l'actionneur central est pilotable par une tension de contrôle ($V_{ctrl}$) ; on peut considérer que la haute tension $V_{cycle}$ délivrée par cette alimentation haute tension $50_3$ est directement proportionnelle à la valeur de cette tension de contrôle. On comprend alors que le circuit de commande $52_3$ permet de piloter la tension appliquée à l'actionneur piézoélectrique central. Il permet donc de commander le déplacement de la membrane centrale $22_3$, selon l'équation explicitée ci-dessus.

Un capteur 54 est couplé à la pompe. Ce capteur permet de mesurer la déformation de la membrane centrale $22_3$, afin de permettre une estimation de la quantité de fluide déplacée par celle-ci.

Ce capteur 54 peut prendre la forme d'une jauge de contrainte déposée sur la membrane ; en variante, ce peut être un capteur optique. Plus généralement, tout capteur permettant de mesurer le déplacement ou la déformation d'une paroi de la chambre centrale $12_3$ convient.

À partir des signaux du capteur 54, un circuit 55 de calcul permet d'estimer le volume débité par la pompe, au cours d'un cycle de cette dernière, par exemple par une méthode d'estimation d'un volume, de type calotte sphérique, mentionnée ci-dessus. Autrement dit, la mesure de la déformation de la membrane centrale $22_3$ permet d'estimer la variation de volume de la chambre centrale $12_3$, ce qui permet l'estimation du volume élémentaire délivré au cours d'un cycle. Le circuit 55 fournit en fait une tension dépendante de ce volume élémentaire, de préférence par une relation de proportionnalité. La sortie de ce circuit 55 est connectée à une 1ère voie d'entrée d'un comparateur 56, qui reçoit également un signal de consigne ($V_D$) sur sa 2ème voie d'entrée. Ce signal de consigne représente le volume élémentaire de fluide à délivrer au cours d'un cycle.

Enfin, un circuit 58 de calcul élabore la tension de commande($V_{ctrl}$) du générateur HT $50_3$ à partir de la sortie du comparateur 56.

Ainsi, à partir :

- du signal délivré par le capteur 54, au cours d'un cycle, ce signal dépendant la déformation de la membrane $22_3$,

- d'un signal de consigne ($V_D$), représentant le volume élémentaire de fluide à délivrer au cours d'un cycle,

le circuit de commande 55, 56, 58, $50_3$ détermine, puis applique, un signal de commande à l'alimentation haute tension $50_3$ de l'actionneur central, et donc un signal de commande de l'actionneur central $51_3$ (en fait, un signal de commande du circuit de commande $52_3$ de ce dernier). Le signal de commande de l'alimentation haute tension est fonction du volume du fluide débité au cours du cycle.

Autrement dit, le circuit de commande, associé à l'actuateur piézoélectrique central, reçoit un signal du générateur $50_3$ qui est fonction du volume estimé et de sa comparaison au volume de consigne $V_D$.

Un autre mode de réalisation est illustré en figure 4, sur laquelle, comme sur les figures suivantes, des références numériques identiques à celles des autres figures y désignent des mêmes éléments.

La quantité de fluide délivré au cours de chaque cycle est ici déterminée par un débitmètre 60, disposé en amont de la pompe, mais de préférence en aval de la pompe, ou intégré dans la pompe. Un exemple d'intégration d'un débitmètre dans une pompe telle que décrite ci-dessus, est donné dans le document WO2012/146753. Le signal de ce débitmètre est intégré par des moyens 62 formant intégrateur, de façon à déterminer la quantité de fluide délivré au cours d'un cycle.

Si le débitmètre 60 produit un signal numérique, les moyens d'asservissement de l'alimentation haute tension $50_3$ sont, dans ce cas, des moyens d'asservissement numérique.

L'estimation du volume élémentaire débité par la pompe, au cours de chaque cycle est obtenue par une intégration du signal du débitmètre au cours d'un cycle de la pompe. Le comparateur 56 joue ici le même rôle que dans le cas de la figure 3, avec une comparaison, là encore, à un signal de consigne ($V_D$) représentant le volume élémentaire de fluide à délivrer au cours d'un cycle. Est donc produit, ici aussi, un signal de commande à l'alimentation haute tension $50_3$ de l'actionneur central, et donc un signal de commande de l'actionneur central $51_3$ (en fait, un signal de commande du circuit de commande $52_3$ de ce dernier), en fonction du volume du fluide débité au cours du cycle.

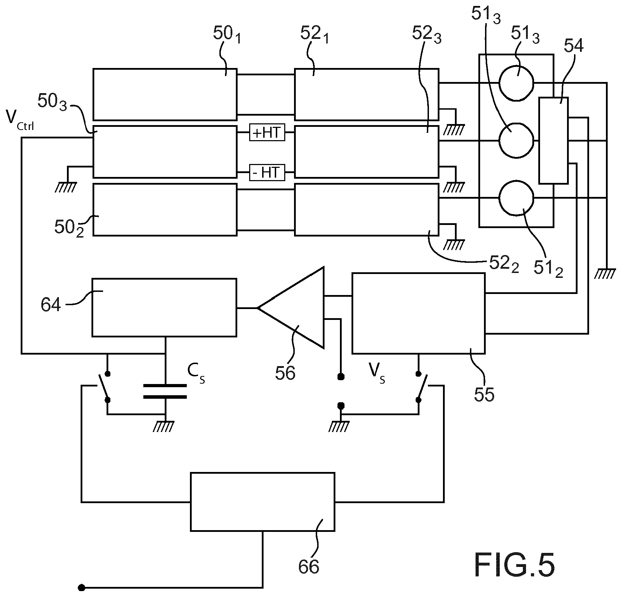

Une variante est représentée en figure 5.

La sortie du comparateur 56 commande un générateur de rampe 64, qui va permettre de piloter l'alimentation haute tension $50_3$ associée à l'actionneur $51_3$ de la membrane centrale $12_3$.

Dans ce mode de réalisation, un générateur de courant 64 pilotable charge une capacité Cs, pendant la phase où le signal de sortie du comparateur 56 est à l'état « bas » (la sortie de l'estimateur 55 de volume est alors inférieure à la consigne). Le générateur est inactif lorsque le comparateur 56 bascule dans l'autre état.

Tant que le volume délivré est inférieur à la consigne $V_s$ (le signal de sortie du comparateur 56 est alors à l'état bas), la tension de contrôle $V_{ctrl}$ délivrée aux moyens d'alimentation $50_3$ augmente, commandant le circuit de commande $52_3$ de manière à assurer un déplacement de l'actionneur correspondant $51_3$. Cela entraîne une déformation progressive de la membrane centrale. Parallèlement, la capacité $C_s$ se charge.

Dès que le volume attendu $V_s$ est atteint, la tension $V_{ctrl}$ délivrée aux moyens $50_3$ reste constante jusqu'à la fin du cycle, du fait de la tension aux bornes de la capacité $C_s$. La membrane centrale $21_3$ s'immobilise, avant d'être réinitialisée à une position initiale à la fin du cycle.

En chaque début de cycle de production d'un incrément de volume, les moyens 55 d'estimation du volume et la capacité Cs sont réinitialisés par des moyens de réinitialisation 66.

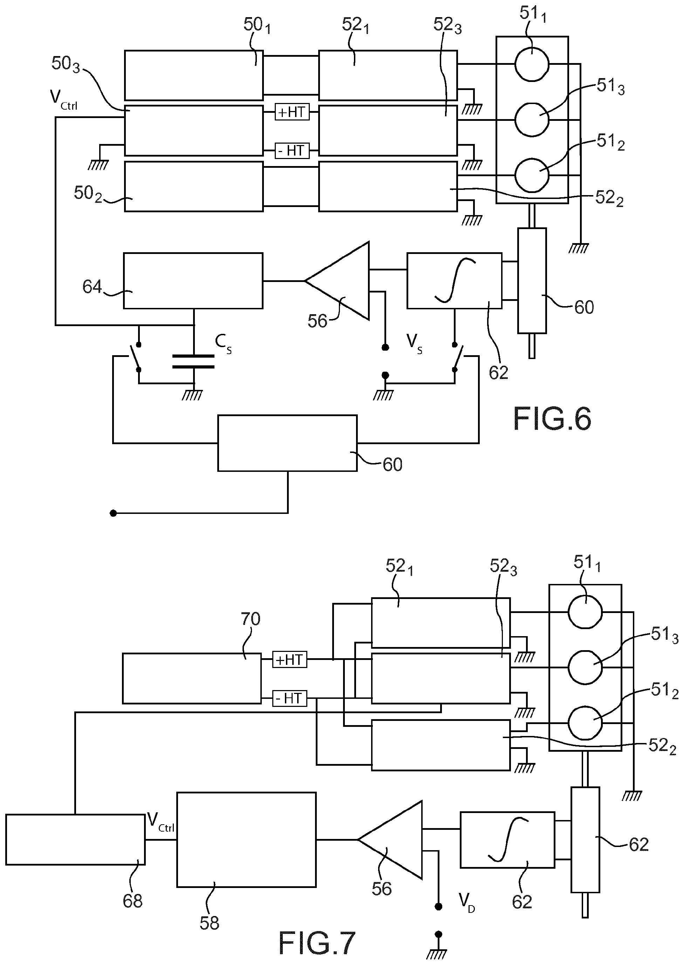

Une variante de ce mode de réalisation, dans le cas où l'on met en œuvre un débitmètre pour l'estimation du volume, est illustrée en figure 6.

En sortie de pompe, le débitmètre 60 fournit un signal à l'intégrateur 62, qui alimente une voie d'entrée du comparateur 56. La sortie de ce dernier commande le générateur de courant 64, qui fonctionne de la manière expliquée ci-dessus.

Encore un autre mode de réalisation est illustré en figure 7.

Ici, on exploite le fait que l'actionneur piézoélectrique de la membrane se comporte, d'un point de vue électrique, comme un circuit série RC, présentant une constante de temps élevée. Aussi, afin de déformer la membrane, on ne module pas la tension aux bornes de l'actionneur, mais on lui applique des impulsions de tension, par exemple sous la forme de créneaux. Plus la fréquence des impulsions est élevée, plus la membrane se déforme. Autrement dit, pour piloter la déformation de la membrane, on ne module pas la haute tension commandant l'actionneur piézoélectrique, mais la fréquence de sa tension d'alimentation. Cette variante permet d'éviter de moduler l'amplitude de la haute tension d'alimentation $50_3$ de l'actionneur piézoélectrique. Dans ce cas, on peut utiliser une alimentation haute tension 70 dont la tension de sortie est fixée, et non variable comme dans les précédents modes de réalisation, et qui va alimenter chacun des circuits de commutation $52_1$ - $52_3$.

Un circuit de modulation 68 va permettre de commander le circuit de commande de l'actionneur $51_3$, dont le rapport cyclique va être modulé. La modulation peut être de type PWM (« Pulse Width Modulation » ou modulation de la largeur d'impulsion).

La fréquence de fonctionnement normal de la pompe étant de quelques dizaines de Hertz, la modulation ci-dessus peut être réalisée à une fréquence de quelques dizaines de kilo Hertz, par exemple entre 10 kHz et 50 kHz, voire jusqu'à 500 kHz.

Là encore, en sortie de pompe, le débitmètre 60 fournit un signal à l'intégrateur 62, qui alimente une voie d'entrée du comparateur 56. Après comparaison avec la consigne $V_D$, la sortie de ce dernier est exploitée par le circuit du calcul 58 qui commande le circuit de modulation 68.

Le même principe de modulation de fréquence peut être mis en œuvre sur les autres configurations qui ont été décrites ci-dessus, en particulier celles qui mettent en œuvre un capteur d'un type autre que le débitmètre cité dans cet exemple.

Selon l'invention, l'actionneur commandant la pompe, reçoit, au cours d'un cycle, un signal de commande dépendant du volume de liquide délivré par la pompe au cours de ce même cycle. Le signal de commande de l'actionneur, au cours d'un cycle, dépend donc de l'évolution du signal du capteur au cours du même cycle, et d'un signal de consigne.

Notons que, quel que soit le mode de réalisation, on peut disposer un moyenneur entre l'intégrateur 62 (ou le circuit 55 d'estimation du volume débité par la pompe) et le comparateur 56. Le moyenneur effectue une moyenne, par exemple une moyenne glissante, de la quantité de fluide délivré au cours des N derniers cycles, N étant un entier supérieur à 2, et notamment compris entre 2 et 20. Aussi, le comparateur réalise une comparaison de cette valeur moyenne à la valeur du signal de consigne $V_D$.

## Revendications

**1.** Système pour délivrer un liquide comportant :

     - une pompe péristaltique (1), fonctionnant par cycle, de telle sorte qu'un volume élémentaire de liquide est délivré au cours de chaque cycle,
     - au moins un actionneur ($51_1$, $51_2$, $51_3$) apte à provoquer la délivrance d'un volume de liquide de la pompe, au cours d'un cycle,
     - un circuit de commande (55, 56, 58, 62), pour appliquer un signal de commande à l'actionneur, de telle sorte que le volume délivré au cours d'un cycle dépend de ce signal appliqué à l'actionneur,

**caractérisé en ce qu'**il comporte :

     - un capteur (60), comportant un débitmètre, associé à des moyens (62) pour intégrer le signal de ce débitmètre, de façon à générer ainsi un signal représentatif du volume délivré au cours d'un cycle ;
     - le circuit de commande (55, 56, 58, 62) déterminant un signal de commande en fonction dudit signal du capteur, représentatif du volume délivré au cours d'un cycle, et d'un signal de consigne ($V_D$, $V_S$).

**2.** Système selon la revendication 1, dans lequel la pompe comporte une chambre centrale ($12_3$) dont le volume peut varier sous l'effet de la déformation ou du déplacement d'une de ses parois, cette variation entraînant la délivrance dudit volume élémentaire.

**3.** Système selon la revendication 2, comportant 2 vannes ($12_i$, $12_2$), disposées de part et d'autre de ladite chambre centrale ($12_3$).

**4.** Dispositif selon l'une des revendications 2 ou 3, ladite chambre centrale ($12_3$) comportant une membrane ($22_3$), dont la déformation est apte à faire varier le volume de ladite chambre centrale, et dont le déplacement est commandé par un desdits actionneurs ($51_3$).

**5.** Système selon l'une des revendications précédentes, le capteur (60) étant apte à délivrer un signal représentant la quantité moyenne de liquide délivrée au cours d'un cycle, ainsi qu'au cours d'un ou plusieurs cycles précédent ledit cycle.

**6.** Système selon l'une des revendications précédentes, le signal de commande étant, tant que la quantité de liquide délivrée est inférieure au signal de consigne, une fonction croissante en fonction du temps.

**7.** Système selon l'une des revendications 1 à 6, l'actionneur ($51_3$) étant piézoélectrique, le système comportant en outre une alimentation en tension ($50_3$) apte à piloter ledit actionneur, cette alimentation étant apte à être commandée par ledit signal de commande.

**8.** Système selon la revendication précédente, le signal de commande étant un signal de modulation de l'amplitude de ladite tension.

**9.** Système selon la revendication 7, le signal de commande étant un signal de modulation de la fréquence de ladite tension.

## Patentansprüche

**1.** System zum Zuführen einer Flüssigkeit, umfassend:

     - eine Peristaltikpumpe (1), die in Zyklen derart arbeitet, dass im Verlauf jedes Zyklus ein Elementarvolumen von Flüssigkeit zugeführt wird,
     - wenigstens ein Betätigungselement ($51_1$, $51_2$, $51_3$), das dazu ausgelegt ist, die Zuführung eines Flüssigkeitsvolumens der Pumpe im Verlauf eines Zyklus zu bewirken,
     - eine Steuerschaltung (55, 56, 58, 62) zum Anlegen eines Steuersignals an das Betätigungselement derart, dass das im Verlauf eines Zyklus zugeführte Volumen von diesem an das Betätigungselement angelegten Signal abhängt,

**dadurch gekennzeichnet, dass** es umfasst:

- einen Sensor (60), der einen Durchflussmesser umfasst, der Mitteln (62) zum Integrieren des Signals dieses Durchflussmessers zugeordnet ist, derart, dass auf diese Weise ein Signal generiert wird, das repräsentativ ist für das im Verlauf eines Zyklus zugeführte Volumen;
- wobei die Steuerschaltung (55, 56, 58, 62) ein Steuersignal als Funktion des Signals von dem Sensor bestimmt, das für das Volumen repräsentativ ist, das im Verlauf eines Zyklus zugeführt wird, sowie eines Sollwertsignals ($V_D$, $V_S$).

2. System nach Anspruch 1, bei dem die Pumpe eine zentrale Kammer ($12_3$) umfasst, deren Volumen unter der Einwirkung der Verformung oder der Verlagerung einer ihrer Wände variieren kann, wobei diese Variation die Zuführung des Elementarvolumens auslöst.

3. System nach Anspruch 2, umfassend zwei Ventile ($12_1$, $12_2$), die auf beiden Seiten der zentralen Kammer ($12_3$) angeordnet sind.

4. Vorrichtung nach einem der Ansprüche 2 oder 3, wobei die zentrale Kammer ($12_3$) eine Membran ($22_3$) umfasst, deren Verformung dazu ausgelegt ist, das Volumen der zentralen Kammer variieren zu lassen, und deren Verlagerung durch eines der Betätigungselemente ($51_3$) gesteuert wird.

5. System nach einem der vorhergehenden Ansprüche, wobei der Sensor (60) dazu ausgelegt ist, ein Signal zu liefern, das die mittlere Menge von Flüssigkeit repräsentiert, die im Verlauf eines Zyklus zugeführt wird, sowie im Verlauf eines oder mehrerer Zyklen, die dem Zyklus vorhergehen.

6. System nach einem der vorhergehenden Ansprüche, wobei das Steuersignal dann, wenn die zugeführte Flüssigkeitsmenge kleiner als das Sollwertsignal ist, eine Funktion ist, die als Funktion der Zeit zunimmt.

7. System nach einem der Ansprüche 1 bis 6, wobei das Betätigungselement ($51_3$) piezoelektrisch ist, wobei das System ferner eine Spannungsversorgung ($50_3$) umfasst, die dazu ausgelegt ist, das Betätigungselement zu steuern, wobei diese Versorgung dazu ausgelegt ist, durch das Steuersignal gesteuert zu werden.

8. System nach dem vorhergehenden Anspruch, wobei das Steuersignal ein Signal zur Modulation der Amplitude der Spannung ist.

9. System nach Anspruch 7, wobei das Steuersignal ein Signal zur Modulation der Frequenz der Spannung ist.

**Claims**

1. A system for delivering a liquid including:

- a peristaltic pump (1), operating on a cycle basis, so that a liquid elementary volume is delivered during each cycle,
- at least one actuator ($51_1$, $51_2$, $51_3$) able to cause a liquid volume to be delivered from the pump, during one cycle,
- a control circuit (55, 56, 58, 62), to apply a control signal to the actuator, so that the volume delivered during one cycle depends on this signal applied to the actuator,

**characterised in that** it includes:

- a sensor (60), including a flowmeter, associated with means (62) for integrating the signal of this flowmeter, so as to thus generate a signal representative of the volume delivered during one cycle;
- the control circuit (55, 56, 58, 62) determining a control signal as a function of said signal from the sensor, representative of the volume delivered during one cycle, and of a set point signal ($V_D$, $V_S$).

2. The system according to claim 1, wherein the pump includes a central chamber ($12_3$) the volume of which can vary under the effect of one of its walls being deformed or displaced, this variation resulting in delivering said elementary volume.

3. The system according to claim 2, including 2 valves ($12_1$, $12_2$), disposed on either side of said central chamber ($12_3$).

4. The device according to one of claims 2 or 3, said central chamber ($12_3$) including a membrane ($22_3$), the deformation of which is able to vary the volume of said central chamber, and the displacement of which is controlled by one of said actuators ($51_3$).

5. The system according to one of the previous claims, the sensor (60) being able to deliver a signal representing the mean liquid quantity delivered during one cycle, as well as during one or more cycles preceding said cycle.

6. The system according to one of the previous claims, the control signal being, as long as the liquid quantity delivered is lower than the set point signal, a time increasing function.

**7.** The system according to one of claims 1 to 6, the actuator ($51_3$) being piezoelectric, the system further including a voltage power supply ($50_3$) able to drive said actuator, this power supply being able to be controlled by said control signal.

**8.** The system according to the previous claim, the control signal being an amplitude modulation signal for said voltage.

**9.** The system according to claim 7, the control signal being a frequency modulation signal for said voltage.

FIG.1A

FIG.1B

FIG.1C

FIG.1D

FIG.1E

FIG.1F

FIG.2

FIG.3

FIG.4

FIG.5

FIG.6

FIG.7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2011058140 A
- FR 2989590 A1
- WO 2013160312 A
- WO 2012146753 A